# EUROPÄISCHE PATENTSCHRIFT

(11) **EP 2 802 297 B1**
(45) Veröffentlichungstag und Bekanntmachung des Hinweises auf die Patenterteilung: **06.01.2016**
(21) Anmeldenummer: 13709059.3
(22) Anmeldetag: 19.02.2013
(51) Int. Cl.: A61F 2/95

(54) **FREISETZVORRICHTUNG ZUM BETÄTIGEN EINES EINFÜHRKATHETERS**
RELEASE DEVICE FOR ACTUATING A DELIVERY CATHETER
DISPOSITIF DE LIBÉRATION DESTINÉ À ACTIONNER UN CATHÉTER D'INTRODUCTION

(30) Priorität: 12.03.2012 DE 202012002563 U
(43) Veröffentlichungstag der Anmeldung: 19.11.2014
(73) Patentinhaber: Variomed AG, 9496 Balzers (LI)
(72) Erfinder: ROTH, Michael, 78315 Radolfzell am Bodensee (DE); PREISER, Jürgen, 79761 Waldshut (DE)
(74) Vertreter: Manitz, Finsterwald & Partner GbR
(86) Internationale Anmeldenummer: PCT/EP2013/053227
(87) Internationale Veröffentlichungsnummer: WO 2013/135462

(56) Entgegenhaltungen:
- WO-A1-00/02503
- WO-A1-97/17899
- US-A1- 2007 156 225

## Beschreibung

Die vorliegende Erfindung betrifft eine Freisetzvorrichtung zum Betätigen eines Einführkatheters, welcher eine Außenhülle und einen in der Außenhülle verschiebbar angeordneten Mandrin umfasst, mit einer länglichen Handhabe, einer an der Handhabe angeordneten Befestigungsaufnahme, welche dazu ausgelegt ist, ein proximales Endstück des Mandrins relativ zur Längsachse der Handhabe unverschieblich an der Handhabe festzulegen, und einer an der Handhabe ausgebildeten Führung, welche dazu ausgelegt ist, ein am proximalen Endbereich der Außenhülle angeordnetes Betätigungselement in Bezug auf die Längsachse der Handhabe und auf den Mandrin verschieblich aufzunehmen.

Die Positionsangaben "distal" und "proximal" beziehen sich jeweils auf eine Bedienperson der Freisetzvorrichtung bzw. des Einführkatheters.

Unter einem Einführkatheter wird eine Vorrichtung zum Einführen eines Stents, eines Drainagerohrs oder dergleichen in Hohlorgane, insbesondere in Blutgefäße, Harnleiter, Speiseröhren oder Gallenwege verstanden. Bei einem derartigen Einführkatheter ist der Stent in komprimiertem Zustand auf das distale Ende des Mandrins aufgeschoben und von dem distalen Ende der Außenhülle überdeckt. Der mit dem Stent versehene Einführkatheter wird in das gewünschte Hohlorgan so weit eingeführt, bis der Stent an der gewünschten Position zu liegen kommt.

US 2007/0156225 A1 beschreibt einen Einführkatheter mit einem Kontrollmechanismus, der mit der Außenhülle und dem Mandrin verbunden ist.

Zum Freisetzen des Stents werden die aus dem Körper des Patienten herausragenden proximalen Enden des Mandrins und der Außenhülle so weit gegeneinander verschoben, bis die Außenhülle den Stent vollständig freigibt, so dass dieser expandieren und sich an die Innenwandung des Hohlorgans anlegen kann. Dabei können sowohl selbstexpandierende Stents verwendet werden, die beispielsweise aus einem sogenannten MemoryMetall bestehen, als auch Stents, die beispielsweise mittels Ballondilatation expandiert werden.

Problematisch ist dabei, dass diese Relativverschiebung sowohl durch ein Zurückziehen der Außenhülle als auch durch körpereinwärts gerichtetes Vorwärtsschieben des Mandrins und insbesondere auch durch eine Kombination dieser beiden Bewegungen erfolgen kann. Eine Vorwärtsbewegung des Mandrins führt jedoch zu einer Positionsänderung des innerhalb des Hohlorgans platzierten Stents, so dass dieser beim Freisetzen aus seiner gewünschten Position wegbewegt wird.

Mit Hilfe einer eingangs genannten, gattungsgemäßen Freisetzvorrichtung ist es möglich, dieses Problem zu vermeiden und den Stent sicher an der gewünschten Stelle innerhalb des Hohlorgans zu positionieren. Die Handhabe wird dabei ortsfest in Bezug auf den Körper des Patienten gelagert und die Außenhülle durch ein Verschieben des Betätigungselements zum Freisetzen des Stents körperauswärts zurückgezogen, so dass der Stent an der korrekten Position freigesetzt wird.

Da jedoch die einzuführenden Stents unterschiedliche Längen aufweisen können und damit auch unterschiedlich lange Verschiebewege der Auβenhülle bzw. des Betätigungselements erforderlich sind, muss für jede Stentlänge eine entsprechende Handhabe vorgesehen werden, bei welcher die Länge der Führung an die Länge des Stents bzw. den Verschiebeweg der Außenhülle angepasst ist. Da somit die Bereitstellung einer Vielzahl von unterschiedlichen Ausführungen der Freisetzvorrichtung erforderlich ist, erhöhen sich die Fertigungs- und Logistikkosten.

Es ist daher eine Aufgabe der Erfindung, eine Freisetzvorrichtung der eingangs genannten Art zu schaffen, welche universell für Einführkatheter mit unterschiedlichen Stentlängen verwendbar ist. Dabei soll die Freisetzvorrichtung möglichst einfach zu bedienen sein, so dass Fehlbedienungen bei der Positionierung praktisch ausgeschlossen sind.

Die Lösung der Aufgabe erfolgt durch die Merkmale des Anspruchs 1 und insbesondere dadurch, dass die Handhabe ein längliches Basisteil und ein mit dem Basisteil verbundenes, entlang der Längsachse des Basisteils verstellbares längliches Verstellteil umfasst, wobei das Verstellteil zwischen einem Justierzustand, in welchem das Verstellteil für eine Längenänderung der Führung axial verschieblich an dem Basisteil gehalten ist, und einem Arretierzustand, in welchem das Verstellteil an dem Basisteil zumindest axial arretiert ist, umstellbar ist. Bei der erfindungsgemäβen Freisetzvorrichtung erstreckt sich in der Regel die Führung über das Basisteil und das Verstellteil. Durch ein Verändern der Relativposition von Basisteil und Verstellteil kann die Länge der Führung individuell an die Erfordernisse des jeweils verwendeten Einführkatheters, d.h. an die Länge des im Einführkatheter angeordneten Stents angepasst werden. Durch die Länge der Führung wird nämlich letztlich der Verstellweg des Betätigungselements und damit auch der Verstellweg der Außenhülle begrenzt.

Bevorzugt ist die Befestigungsaufnahme an dem Verstellteil vorgesehen. Das Basisteil kann an einem der Befestigungsaufnahme gegenüberliegenden Ende eine Führungshülse für die Außenhülle des Einführkatheters aufweisen. Grundsätzlich kann dies jedoch auch umgekehrt der Fall sein, d.h. die Befestigungsaufnahme ist an dem Basisteil vorgesehen, während die Führungshülse an dem Verstellteil angeordnet sein kann.

Gemäß einer bevorzugten Ausführungsform ist das Verstellteil durch Verdrehen um seine Längsachse zwischen dem Justierzustand und dem Arretierzustand umstellbar. Dadurch sind der Justierzustand und der Arretierzustand eindeutig voneinander unterscheidbar. Darüber hinaus ist für die Umstellung des Verstellteils auf diese Weise kein separates Element, sondern lediglich eine Drehbewegung erforderlich.

Bevorzugt hintergreifen das Verstellteil und das Basisteil in dem Arretierzustand des Verstellteils, nicht jedoch in dem Justierzustand des Verstellteils einander formschlüssig. Dadurch wird eine besonders zuverlässige Arretierung von Basisteil und Verstellteil im Arretierzustand erreicht.

Gemäß einer vorteilhaften Ausführungsform weist das Basisteil eine entlang seiner Längsachse verlaufende Verstellteilaufnahme auf, in welcher das Verstellteil gehalten ist, wobei die Verstellteilaufnahme und das Verstellteil bevorzugt die Grundform eines Zylinders aufweisen. Eine derartige teleskopartige Ausgestaltung lässt sich fertigungstechnisch einfach herstellen und gewährleistet dennoch eine stabile Verbindung zwischen Basisteil und Verstellteil im Arretierzustand.

Gemäß einer weiteren bevorzugten Ausführungsform weist die Verstellteilaufnahme mehrere insbesondere quer verlaufende, axial beabstandete Sicherungsausnehmungen auf und das Verstellteil weist an seiner Außenseite wenigstens einen Sicherungsvorsprung auf, wobei der Sicherungsvorsprung in dem Arretierzustand des Verstellteils mit einer der Sicherungsausnehmungen in Eingriff steht. Die Sicherungsausnehmungen können durch entsprechende Eintiefungen in der Verstellteilaufnahme, d.h. einer Wandung des Basisteils, aber auch durch Zwischenräume zwischen voneinander beabstandeten Ansätzen oder Lamellen, welche in der Verstellteilaufnahme vorgesehen sind, gebildet sein. Es versteht sich, dass auch eine umgekehrte Anordnung, bei welcher die Sicherungsausnehmungen am Verstellteil und der Sicherungsvorsprung in der Verstellteilaufnahme des Basisteils ausgebildet sind, möglich ist. Auch kann beispielsweise nur eine Sicherungsausnehmung vorgesehen sein, die mit mehreren Sicherungsvorsprüngen zusammenwirken kann. Durch den Abstand der Sicherungsausnehmungen bzw. der Sicherungsvorsprünge kann ein Verstellraster für die Längenänderung der Führung vorgegeben werden.

Grundsätzlich bestehen auch andere Möglichkeiten, das Verstellteil am Basisteil zu arretieren. So kann beispielsweise eine zwischen den beiden Teilen wirksame Verrastung vorgesehen sein. Der Justierzustand wird dann durch eine entsprechende Kraftausübung, die die Verrastung überwindet, hergestellt. Weiterhin kann die Arretierung mithilfe eines Arretierelements, etwa einer Schraube, eines Stifts oder eines Splints, erfolgen, das in jeweilige seitlich am Basiselement und am Verstellteil vorgesehene, miteinander fluchtende Öffnungen eingeführt wird. Ferner kann die Arretierung auch mit Hilfe einer seitlich am Basisteil angeordneten Klemmschraube erfolgen, die im Arretierzustand eine Reibkraft auf das Verstellteil ausübt.

Vorzugsweise ist das Verstellteil in dem Arretierzustand und/oder in dem Justierzustand radial an dem Basisteil verrastet. Dadurch wird ein unbeabsichtigtes Verdrehen des Verstellteils verhindert oder zumindest wesentlich erschwert.

Gemäß einer weiteren vorteilhaften Ausführungsform ist das Verstellteil lediglich von dem Justierzustand in den Arretierzustand, nicht jedoch von dem Arretierzustand in den Justierzustand umstellbar. Hierdurch ist es möglich, die Längenanpassung der Handhabe in Abstimmung auf den verwendeten Einführkatheter bereits herstellerseitig vorzunehmen und die derart angepasste Freisetzvorrichtung zusammen mit dem zugehörigen Einführkatheter als eine gebrauchsfertig montierte, manipulationssichere Einheit auszuliefern. Somit ist eine korrekte Längenanpassung durch qualifiziertes Personal gesichert und eine nachträgliche Veränderung der Führungslänge durch den Anwender weitgehend ausgeschlossen.

Bevorzugt weist die Verstellteilaufnahme wenigstens eine entlang ihrer Längsachse verlaufende Rastnut auf und das Verstellteil weist an seiner Außenseite eine Rastnase auf, wobei die Rastnase zumindest in dem Arretierzustand in die Rastnut eingreift, um das Verstellteil an dem Basisteil radial zu verrasten. Es versteht sich, dass in Umkehrung auch die Rastnase an der Verstellaufnahme und die Rastnut an dem Verstellteil vorgesehen sein kann. Wenn die Verstellteilaufnahme wenigstens zwei Rastnuten aufweist, kann zusätzlich auch eine Verrastung in dem Justierzustand vorgesehen sein.

Gemäß einer weiteren vorteilhaften Ausführungsform weist die Verstellteilaufnahme eine erste und eine zweite entlang ihrer Längsachse verlaufende Rastnut auf, wobei die Rastnase in dem Justierzustand mit der ersten Rastnut und in dem Arretierzustand mit der zweiten Rastnut in Eingriff steht, wobei die Rastnase und die erste Rastnut eine jeweilige Anlaufschräge aufweisen, und wobei die Rastnase und die Rastnuten derart zusammenwirken, dass die Rastnase durch ein Umstellen des Verstellteils von dem Justierzustand in den Arretierzustand aufgrund eines Zusammenwirkens der Anlaufschrägen außer Eingriff mit der ersten Rastnut nicht jedoch durch ein Umstellen von dem Arretierzustand in den Justierzustand außer Eingriff mit der zweiten Rastnut gebracht werden kann. Dadurch kann auf einfache Weise das vorstehend erläuterte Überführen des Verstellteils von dem Arretierzustand in den Justierzustand verhindert werden.

Es ist bevorzugt, wenn das Betätigungselement durch ein an dem proximalen Endbereich der Außenhülle vorgesehenes Anschlussstück oder durch ein mit dem proximalen Endbereich der Außenhülle koppelbares, separates Element gebildet ist. Im ersten Fall kann beispielsweise ein seitlich vom Anschlussstück abstehendes Abzweigrohr, über das Medikamente in den Einführkatheter zugespritzt werden können, als Betätigungselement dienen. Im zweiten Fall können durch das separate Betätigungselement der Bedienkomfort und die Führungspräzision weiter gesteigert werden.

Gemäß einer weiteren vorteilhaften Ausführungsform ist das Betätigungselement drehfest und/oder in axialer Richtung fest mit dem proximalen Endbereich der Außenhülle, insbesondere mit einem an dem proximalen Endbereich der Außenhülle vorgesehenen Anschlussstück, verbunden oder verbindbar, insbesondere verrastet oder verrastbar. Dadurch wird eine zuverlässige Kopplung zwischen dem Betätigungselement und der Außenhülle des Einführkatheters gewährleistet.

Vorzugsweise ist das Betätigungselement zumindest in einer vorgegebenen Axialposition durch Verdrehen um die Längsachse der Handhabung zwischen einer Blockierstellung, in welcher das Betätigungselement in axialer Richtung arretiert ist, und einer Freigabestellung, in welcher das Betätigungselement axial verschieblich in der Führung aufgenommen ist, verstellbar. Die vorgegebene Axialposition entspricht vorzugsweise einer Relativposition von Außenhülle und Mandrin, in welcher die Außenhülle den Stent vollständig überdeckt. In der Blockierstellung wird ein unbeabsichtigtes Freisetzen des Stents wirkungsvoll verhindert. Die axiale Arretierung des Betätigungselements kann beispielsweise durch eine entsprechende Ausgestaltung der Führung nach Art einer Bajonettverriegelung erreicht werden.

Gemäß einer weiteren bevorzugten Ausführungsform weist das Basisteil eine im Wesentlichen plane Auflagefläche auf. Die Auflagefläche dient dazu, die Handhabe ortsfest in Bezug auf den Patienten zu lagern.

Die vorliegende Erfindung bezieht sich ferner auf eine Einführkatheteranordnung, welche einen Einführkatheter mit einer Außenhülle und einen in der Außenhülle verschiebbar angeordneten Mandrin und eine Freisetzvorrichtung zum Betätigen des Einführkatheters nach einer der vorstehend erläuterten Ausführungsformen umfasst.

Weitere vorteilhafte Ausführungsformen der Erfindung sind in den Unteransprüchen, der Beschreibung und den Zeichnungen genannt.

Die Erfindung wird nachfolgend anhand eines Ausführungsbeispiels mit Bezug auf die Zeichnungen erläutert. Es zeigen:
- Fig. 1 bis 4: perspektivische Ansichten einer erfindungsgemäßen Einführkatheteranordnung mit einem Einführkatheter und einer erfindungsgemäßen Freisetzvorrichtung in verschiedenen Verstellpositionen,
- Fig. 5 bis 9: perspektivische Detailansichten, teils geschnitten, von einzelnen Komponenten der Einführkatheteranordnung von Fig. 1 bis 4,
- Fig. 10 bis 15: perspektivische Ansichten der erfindungsgemäßen Einführkatheteranordnung und ihrer Komponenten in verschiedenen Montagezuständen, und
- Fig. 16 und 17: Schnittdarstellungen der erfindungsgemäßen Einführkatheteranordnung in verschiedenen Montagezuständen.

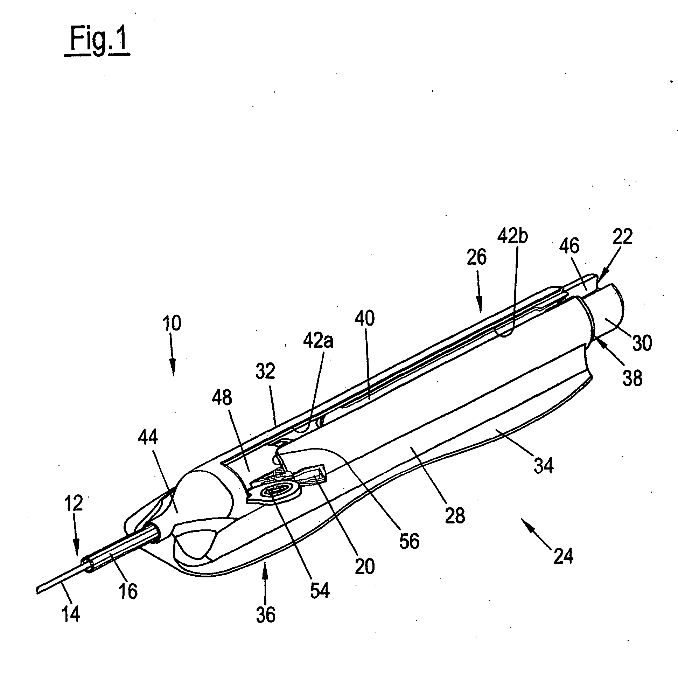

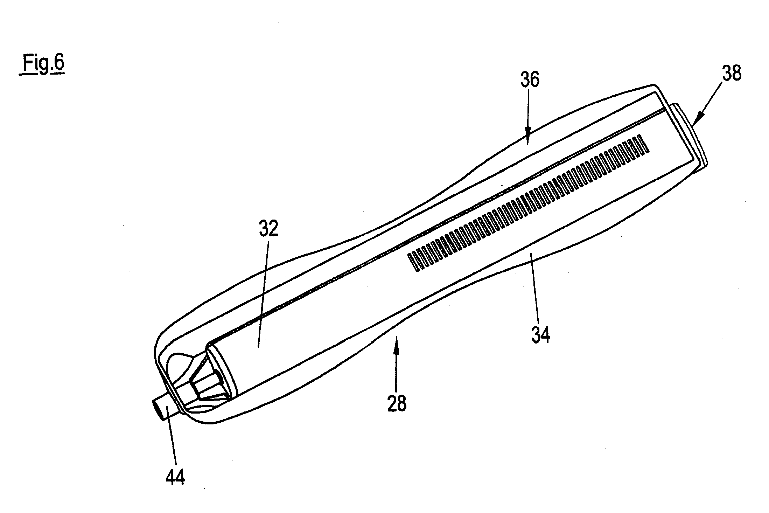

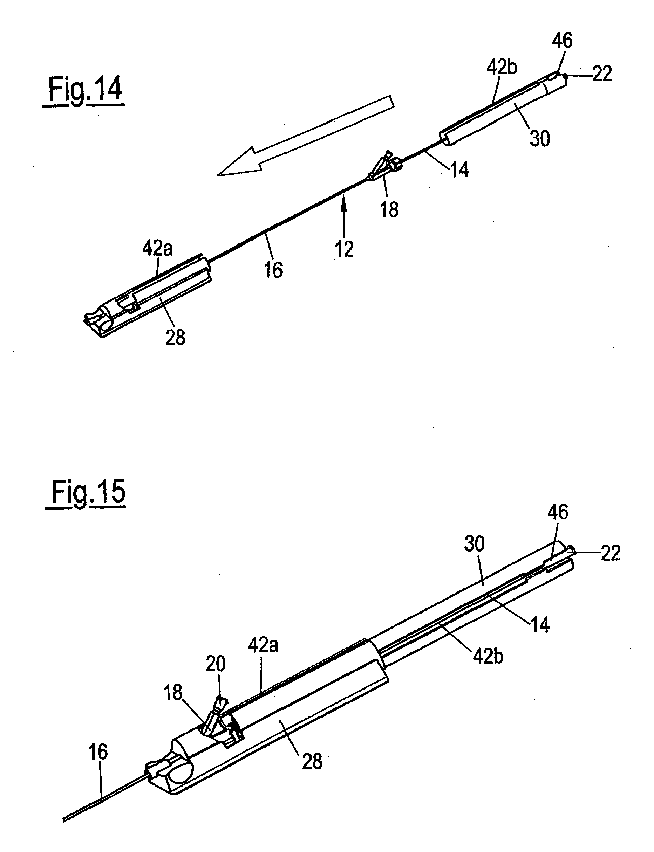

Mit Bezug auf Fig. 1 bis 17 wird zunächst der grundlegende Aufbau einer erfindungsgemäßen Einführkatheteranordnung 10 erläutert.

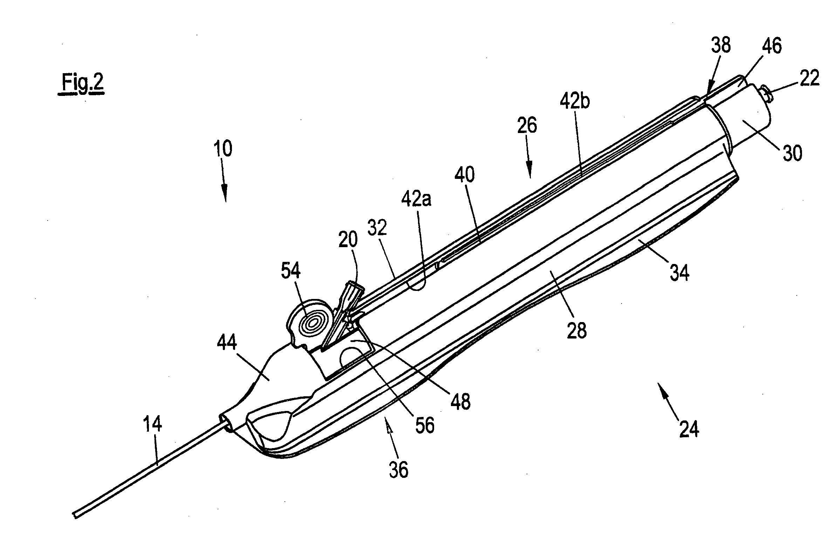

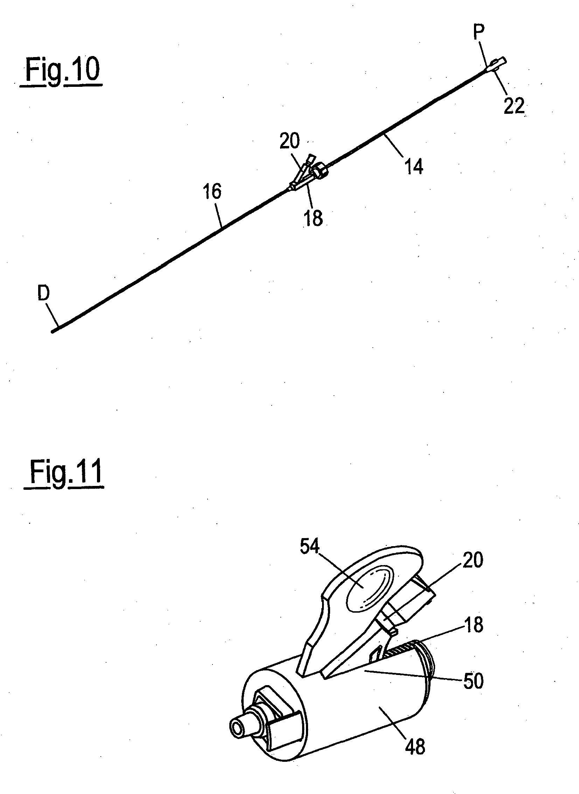

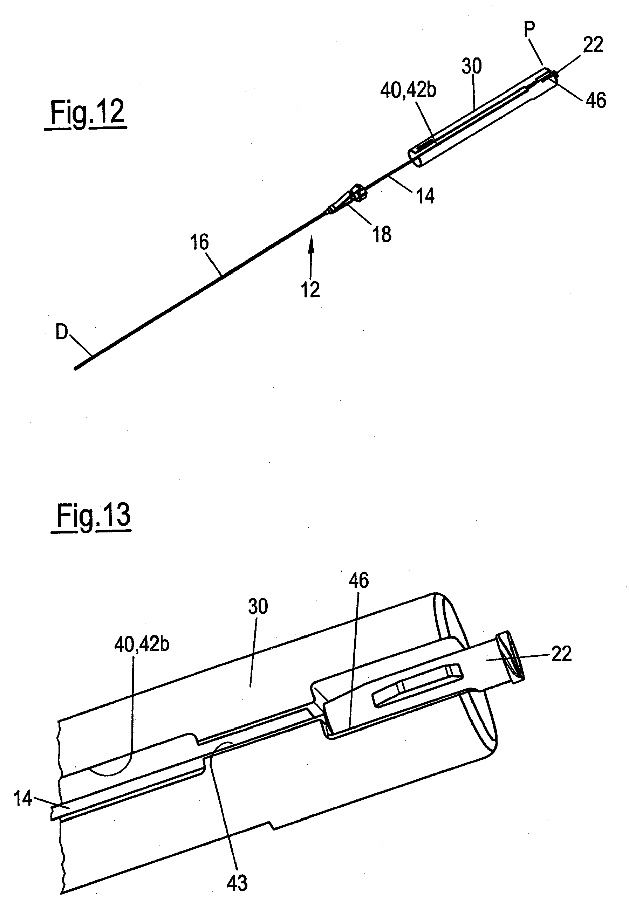

Wie insbesondere in Fig. 1 bis 4 gut zu erkennen ist, umfasst die Einführkatheteranordnung 10 einen an sich bekannten Einführkatheter 12 mit einer Außenhülle 16 und einen in der Außenhülle 16 verschiebbar angeordneten Mandrin 14. Die Außenhülle 16 ist in Fig. 1 nur abschnittsweise und in Fig. 2 bis 4 gar nicht dargestellt. Vollständige Darstellungen des Einführkatheters 12 finden sich insbesondere in Fig. 10 und 12.

Am distalen Ende D des Einführkatheters 12 ist ein Stent angeordnet (nicht dargestellt), welcher sich im komprimierten Zustand zwischen dem Mandrin 14 und der Außenhülle 16 befindet. Durch ein Zurückziehen der Außenhülle 16 kann der Stent in einem Hohlorgan eines Patienten freigesetzt werden und expandieren.

Am proximalen Ende P des Einführkatheters 12 ist ein Nippel 22 an einem Endstück des Mandrins 14 ausgebildet. Am proximalen Endbereich der Außenhülle 16 ist ein Anschlussstück 18 mit einem unter einem Winkel von ca. 45° abstehenden Abzweig 20 vorgesehen.

Die Einführkatheteranordnung 10 umfasst weiterhin eine erfindungsgemäße Freisetzvorrichtung 24, welche am proximalen Ende P des Einführkatheters 12 angeordnet ist.

Die Freisetzvorrichtung 24 weist eine Handhabe 26 auf, welche aus einem länglichen Basisteil 28 und einem längenverstellbar mit dem Basisteil 28 verbundenen länglichen Verstellteil 30 zusammengesetzt ist.

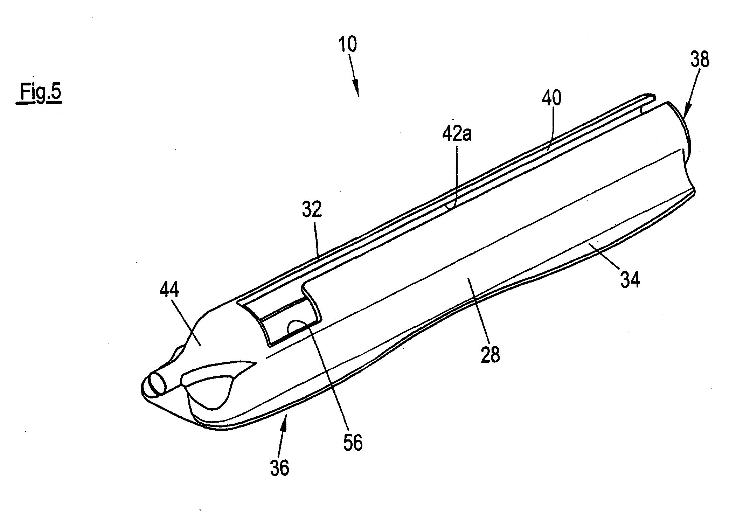

Wie insbesondere in Fig. 5 bis 7 - die nur das Basisteil 28 zeigen - gut zu erkennen ist, weist das Basisteil 28 einen Rohrabschnitt 32 mit hohlzylindrischer Grundform und einen integral am Rohrabschnitt 32 vorgesehenen Rahmenabschnitt 34 auf, wobei der Rahmenabschnitt 34 an der Unterseite der Handhabe 26 eine im Wesentlichen plane Auflagefläche 36 bildet. Das Verstellteil 30, welches ebenfalls eine hohlzylindrische Grundform aufweist, ist in einer im Inneren des Rohrabschnitts 32 ausgebildeten Verstellteilaufnahme 38 aufgenommen, wie es beispielsweise aus den Fig. 1 bis 4 zu erkennen ist. Das Verstellteil 30 ragt aus dem proximalen Ende des Basisteils 28 heraus.

Die Handhabe 26 weist eine schlitzförmige Führung 40 auf, wobei sich ein Teilabschnitt 42a der Führung 40 über das Basisteil 28 und ein anderer Teilabschnitt 42b der Führung 40 über das Verstellteil 30 erstreckt.

Am distalen Ende des Basisteils 28 ist eine trichterartige Führungshülse 44 ausgebildet, durch die der Mandrin 14 mit der ihn umgebenden Auβenhülle 16 des Einführkatheters 12 hindurch geführt ist.

Der Mandrin 14 ist mit Hilfe des Nippels 22, welcher in einer am proximalen Ende des Verstellteils 30 vorgesehenen Befestigungsaufnahme 46 aufgenommen ist, mit dem Verstellteil 30 verbunden, insbesondere verrastet oder verklemmt.

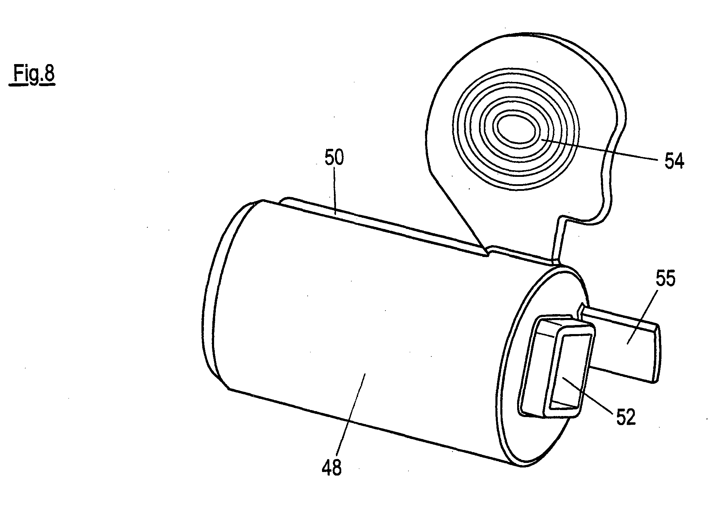

Im Inneren der Handhabe 26 ist ein kappenartiges Betätigungselement 48, das detailliert in Fig. 8 dargestellt ist, verschiebbar angeordnet. Das Betätigungselement 48 kann auf das Anschlussstück 18 des Einführkatheters 12 aufgesteckt werden (vgl. Fig. 11). Als Durchführung für den Mandrin 14 und die Außenhülle 16 weist das Betätigungselement 48 eine stirnseitige Öffnung 52 mit quadratischem Querschnitt auf. Um einen Durchtritt für den Abzweig 20 des Anschlussstücks 18 zu schaffen, ist am Umfang des Betätigungselements 48 ein länglicher Schlitz 50 vorgesehen., In Verlängerung des Schlitzes 50 ist ein radial abstehender Greifabschnitt 54 vorgesehen. Wenn das Betätigungselement 48 in die Handhabe 26 eingesetzt ist, ragen der Greifabschnitt 54 und der Abzweig 20 durch die Führung 40 hindurch nach außen.

Der Teilabschnitt 42a der Führung 40 ist in einem unmittelbar an die Führungshülse 44 angrenzenden Endbereich durch eine rechteckige Aussparung 56 in Umfangsrichtung des Rohrabschnitts 32 erweitert. Die Länge der Aussparung 56 ist so bemessen, dass diese in einer nachfolgend noch näher erläuterten Blockierstellung des Betätigungselements 48 den Greifabschnitt 54 zusammen mit dem Abzweig 20 aufnehmen kann (siehe Fig. 1).

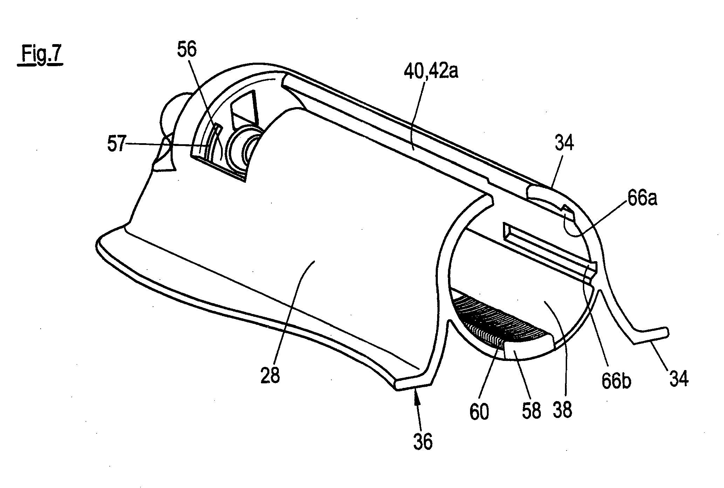

An der Stirnseite des Betätigungselements 48 ist ferner ein Ansatz 55 (Fig. 8) vorgesehen, welcher mit einem am Basisteil 28 vorgesehenen Sicherungsabschnitt 57 (Fig. 7) zusammenwirkt, um das Betätigungselement 48 beispielsweise durch Verrasten oder durch Reibschluss in der Blockierstellung gegen ein unabsichtigtes Verdrehen zu sichern.

Der Teilabschnitt 42b der Führung 40 weist an seinem der Befestigungsaufnahme 46 zugewandten Ende eine Verengung 43 auf, welche als Endanschlag für das Betätigungselement bzw. das Anschlussstück 18 dient.

Im Bereich der Öffnung 52 des Betätigungselements 48 kann eine Rastvorrichtung (nicht dargestellt) vorgesehen sein, mit deren Hilfe das Betätigungselement 48 mit dem Anschlussstück 18 verrastet werden kann. Der rechteckige Querschnitt der Öffnung 52 gewährleistet, dass insbesondere auch solche Anschlussstücke, die keinen Abzweig aufweisen, drehfest mit dem Betätigungselement 48 gekoppelt werden können.

Gemäß einer Abwandlung kann die erfindungsgemäße Freisetzvorrichtung 24 auch ohne ein separates Betätigungselement 48 ausgestaltet sein. In dem Fall übernimmt der Abzweig 20 des Anschlussstücks 18 die Funktion des Betätigungselements. Mit Hilfe des separaten Betätigungselements 48 ist jedoch eine präzisere Führung des Anschlussstücks 18 und eine ergonomischere Handhabung gewährleistet.

Mit Bezug auf Fig. 7, 9, 16 und 17 wird nachfolgend die längenverstellbare Verbindung zwischen dem Basisteil 28 und dem Verstellteil 30 näher erläutert.

In der Verstellteilaufnahme 38 des Basisteils 28 sind mehrere quer verlaufende, in Längsrichtung voneinander beabstandete Lamellen 58 angeordnet, wobei die Zwischenräume zwischen den Lamellen 58 jeweilige Sicherungsausnehmungen 60 definieren (siehe insbesondere Fig. 7). Am Auβenumfang des Verstellteils 30 ist ein ebenfalls quer verlaufender Sicherungsvorsprung 62 vorgesehen, welcher mit den Sicherungsausnehmungen 60 zusammenwirken kann.

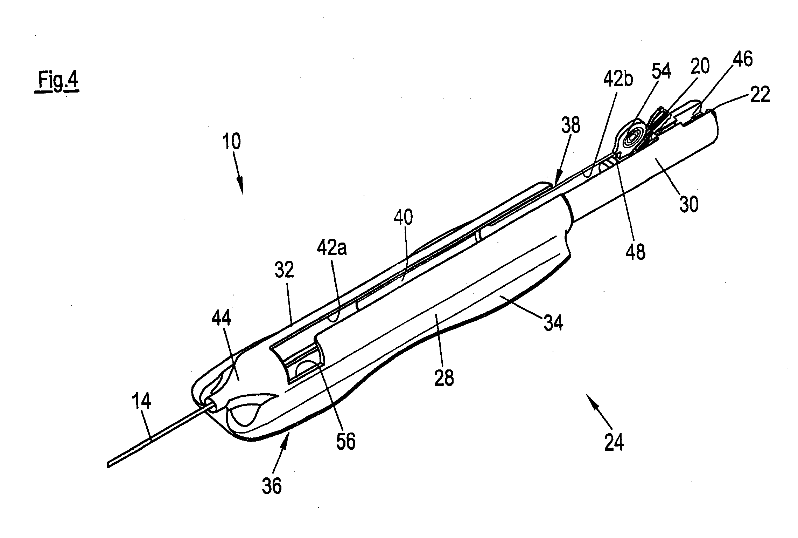

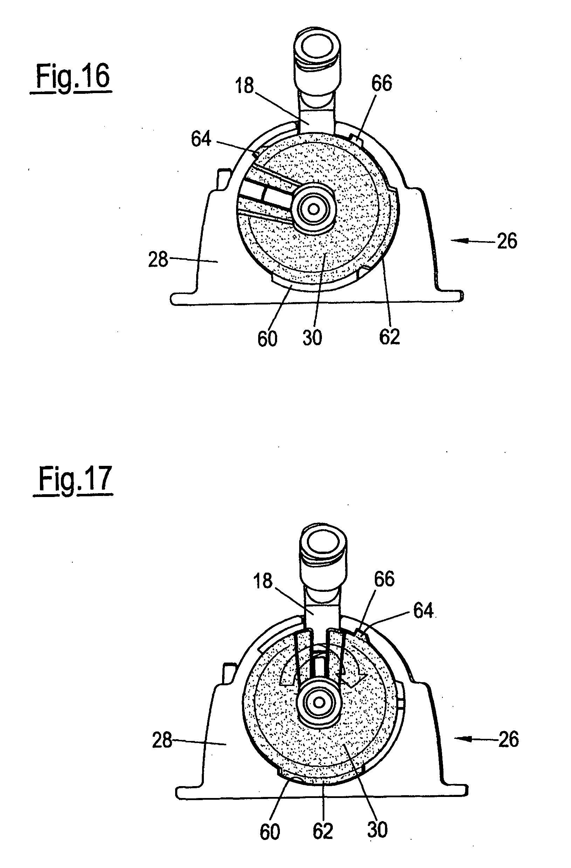

Wenn sich das Verstellteil 30 gemäß Fig. 16 in einem Justierzustand oder einer Justierstellung befindet, greift der Sicherungsvorsprung 62 nicht in eine der Sicherungsausnehmungen 60 ein, so dass das Verstellteil 30 für eine Längenänderung der Führung 40 in axialer Richtung in der Verstellteilaufnahme 38 verschoben werden kann. Auf diese Weise kann die Länge der Führung vor Auslieferung der Einführkatheteranordnung auf die Länge des jeweils spezifisch verwendeten Stents angepasst werden. Beispielsweise sind in Fig. 1 ein an einen kurzen Stent (z.B. 80-100mm) und in Fig. 4 ein an einen langen Stent (z.B. bis zu 250mm) angepasste Freisetzvorrichtungen dargestellt.

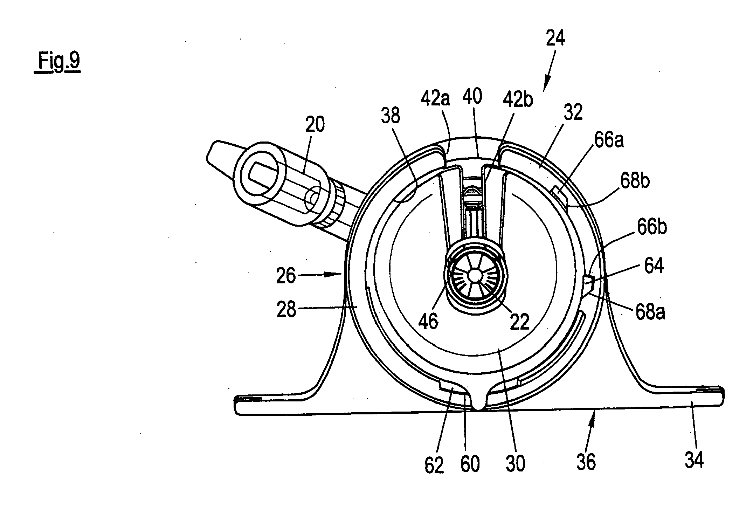

Durch ein Verdrehen des Verstellteils 30 im Uhrzeigersinn kann das Verstellteil 30 von dem Justierzustand (Fig. 16) in einen Arretierzustand oder eine Arretierstellung (Fig. 17) überführt werden. Im Arretierzustand greift der Sicherungsvorsprung 62 in eine der Sicherungsausnehmungen 60 ein, so dass das Verstellteil 30 an dem Basisteil 28 axial arretiert ist (siehe Fig. 9 und 17). Es versteht sich, dass zur Erhöhung der Stabilität anstelle eines einzigen Sicherungsvorsprungs 62 auch mehrere Sicherungsvörsprünge vorgesehen werden können.

Für eine Arretierung in radialer Richtung weist das Verstellteil 30 an seinem Außenumfang eine in radialer Richtung federnde Rastnase 64 auf, welche im Arretierzustand (Fig. 17) in eine in der Verstellteilaufnahme 38 vorgesehene, in Längsrichtung verlaufende Rastnut 66 eingreift.

Gemäß einer Abwandlung, die in Fig. 7 und 9 dargestellt ist, weist die Verstellteilaufnahme 38 anstelle einer einzigen Rastnut zwei in Umfangsrichtung voneinander beabstandete Rastnuten 66a, 66b auf. Die Rastnase 64 steht im Justierzustand mit der Rastnut 66a und im Arretierzustand mit der Rastnut 66b in Eingriff.

Die eine Seite der Rastnase 64 verläuft in radialer Richtung und bildet eine Anschlagfläche, während die gegenüberliegende Seite der Rastnase 64 eine Anlaufschräge 68a aufweist, die im Justierzustand mit einer in der Rastnut 66a ausgebildeten Anlaufschräge 68b derart zusammenwirkt, dass beim Verdrehen des Verstellteils 30 vom Justierzustand in den Arretierzustand die Rastnase 64 entgegen ihrer Vorspannung radial versetzt wird und der Eingriff mit der Rastnut 66a aufgehoben wird. Somit kann das Verstellteil 30 zwar vom Justierzustand in den Arretierzustand überführt werden, nicht jedoch vom Arretierzustand zurück in den Justierzustand, da die radial verlaufenden Anschlagflächen der Rastnut 66b und der Rastnase 64 eine entsprechende Verdrehung des Verstellteils 30 blockieren. Entsprechendes gilt auch für die Ausführung mit der Rastnut 66.

Dadurch wird eine ungewollte Längenverstellung der Handhabe 26 wirksam verhindert. Um dennoch ein Zurückstellen in den Justierzustand zu ermöglichen, kann gemäß einer Abwandlung das Basisteil 28 mit einer Öffnung (nicht dargestellt) im Bereich der Rastnut 66b versehen werden, durch welche beispielsweise mit Hilfe eines Werkzeugs die Rastnase 64 zurückgedrückt und damit außer Eingriff mit der Rastnut 66b gebracht werden kann, so dass ein Verdrehen nunmehr möglich ist.

Mit Bezug auf Fig. 10 bis 17 wird nachfolgend die Montage der Einführkatheteranordnung 10 erläutert.

Der Einführkatheter 12 (Fig. 10) wird zunächst mit seinem distalen Ende D durch die Öffnung 52 des Betätigungselements 48 hindurchgeschoben, bis sich das Anschlussstück 18 im Inneren des Betätigungselements 48 befindet und ggf. mit diesem verrastet (Fig. 11).

In einem nächsten Schritt wird das aus der Außenhülle 16 herausstehende proximale Ende des Mandrins 14 durch die Führung 40 hindurch in das Verstellteil 30 eingelegt und der Nippel 22 mit der Befestigungsaufnahme 46 in Eingriff gebracht (Fig. 12 und 13).

In einem weiteren Schritt wird das distale Ende D des Einführkatheters 12 in das Basisteil 28 eingeführt und durch die Führungshülse 44 hindurchgeschoben. Im Verlauf dieser Verschiebebewegung gelangt zunächst das Betätigungselement 48 (in Fig. 12 bis 17 nicht dargestellt) und das Anschlussstück 18 in den Bereich des Teilabschnitts 42a der Führung 40. Im weiteren Verlauf der Verschiebebewegung wird das Verstellteil 30 in die Verstellaufnahme 38 eingeführt, wobei darauf zu achten ist, dass sich das Basisteil 28 und das Verstellteil 30 in Bezug auf ihre Winkelposition im Justierzustand befinden. Der Einführkatheter 12 wird zusammen mit dem Verstellteil 30 so weit verschoben, bis das Betätigungselement 48 bzw. das Anschlussstück 18 das der Führungshülse 44 benachbarte Ende der Führung 40 erreicht hat.

In einem folgenden Schritt wird das Verstellteil 30 durch Verdrehen gegenüber dem Basisteil 28 vom Justierzustand (Fig. 16) in den Arretierzustand (Fig. 17) überführt.

In einem weiteren Schritt kann der Greifabschnitt 54 des Betätigungselements 48 zusammen mit dem Abzweig 20 des Anschlussstücks 18 ergriffen und in die Aussparung 56 hinein verdreht werden, so dass das Betätigungselement 48 bezüglich seiner Axialposition in der Führung 40 nach Art einer Bajonettverriegelung blockiert ist, um ein versehentliches Betätigen des Einführkatheters 12 und damit ein unbeabsichtigtes Freisetzen des Stents zu verhindern. Die entsprechende Verstellposition der Einführkatheteranordnung 10 ist in Fig. 1 dargestellt. Damit befindet sich die Einführkatheteranordnung 10 in ihrem Auslieferungszustand.

Nachfolgend wird die Bedienung der montierten Einführkatheteranordnung 10 beschrieben.

Das Einführen des Einführkatheters 12 in ein Hohlorgan eines Patienten erfolgt in der in Fig. 1 dargestellten Konfiguration der Einführkatheteranordnung 10. Sobald das distale Ende D des Einführkatheters 12 mit dem Stent die vorgesehene Position im Hohlorgan des Patienten erreicht hat, wird die Handhabe 26 mit ihrer Auflagefläche 36 auf eine gegenüber dem Patienten ortsfeste Unterlage aufgesetzt. Anschließend wird das mit dem Anschlussstück 18 gekoppelte Betätigungselement 48 aus der Aussparung 56 hinausgedreht, so dass es nunmehr frei in der Führung 40 bewegt werden kann (Fig. 2).

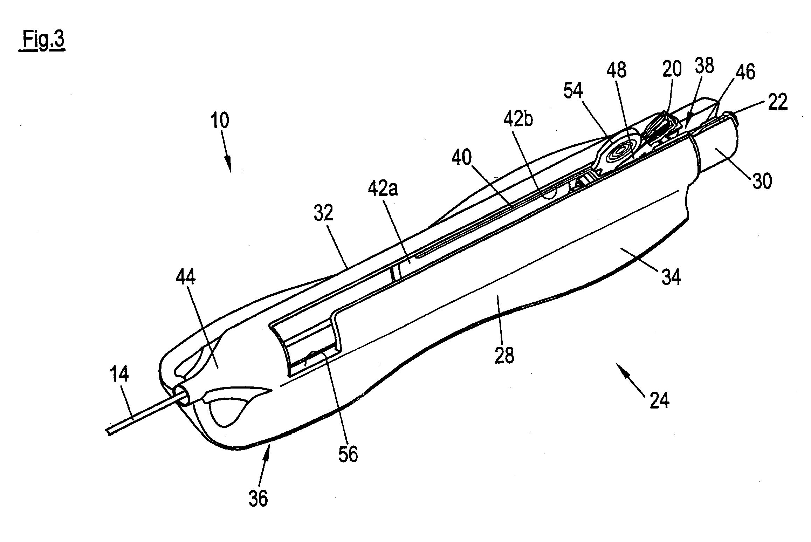

Durch Verschieben des mit dem Anschlussstück 18 gekoppelten Betätigungselements 48 in der Führung 40 in Richtung des proximalen Endes P des Einführkatheters 12 wird die mit dem Anschlussstück 18 verbundene Außenhülle 16 vom distalen Ende D des Einführkatheters 12 zurückgezogen und damit der Stent freigesetzt (Fig. 3). Da der Mandrin 14 über den Nippel 22 und die Befestigungsaufnahme 46 fest mit der ortsfest gelagerten Handhabe 26 verbunden ist, wird eine versehentliche Verschiebung des Mandrins 14 und damit eine unbeabsichtigte Verschiebung des Stents wirkungsvoll vermieden.

In Fig. 4 ist eine Konfiguration der Einführkatheteranordnung 10 dargestellt, welche prinzipiell der Konfiguration von Fig. 3 entspricht. Im Unterschied zu Fig. 3 ist bei der Konfiguration gemäß Fig. 4 das Verstellteil 30 jedoch weniger weit in das Basisteil 28 hinein geschoben. Dadurch vergrößert sich die Länge der Führung 40. Somit kann die Freisetzvorrichtung 24 in der in Fig. 4 dargestellten Konfiguration für längere Stents und damit für Einführkatheter 12 verwendet werden, die im Vergleich zu Fig. 3 einen größeren, zur Freisetzung des Stents erforderlichen Verschiebeweg der Außenhülle 16 relativ zum Mandrin 14 erlauben.

Daher kann die erfindungsgemäße Freisetzvorrichtung 24 universell für Einführkatheter 12 mit unterschiedlichen Stentlängen und daraus resultierenden unterschiedlichen Verschiebewegen der Außenhülle 16 eingesetzt werden. Durch entsprechende Dimensionierung des Basisteils 28 und des Verstellteils 30 können beispielsweise Stentlängen bzw. Verstellwege von etwa 80 mm bis typischerweise 120 mm aber auch bis zu 200 mm oder sogar bis 250 mm abgedeckt werden. Auch kürzere Längen als 80 mm lassen sich realisieren.

### Bezugszeichenliste

- 10: Einführkatheteranordnung
- 12: Einführkatheter
- 14: Mandrin
- 16: Außenhülle
- 18: Anschlussstück
- 20: Abzweig
- 22: Nippel
- 24: Freisetzvorrichtung
- 26: Handhabe
- 28: Basisteil
- 30: Verstellteil
- 32: Rohrabschnitt
- 34: Rahmenabschnitt
- 36: Auflagefläche
- 38: Verstellteilaufnahme
- 40: Führung
- 42a, b: Teilabschnitt
- 43: Verengung
- 44: Führungshülse
- 46: Befestigungsaufnahme
- 48: Betätigungselement
- 50: Schlitz
- 52: Öffnung
- 54: Greifabschnitt
- 55: Ansatz
- 56: Aussparung
- 57: Sicherungsabschnitt
- 58: Lamelle
- 60: Sicherungsausnehmung
- 62: Sicherungsvorsprung
- 64: Rastnase
- 66, 66a, 66b: Rastnut
- 68a, 68b: Anlaufschräge

- D: distales Ende
- P: proximales Ende

## Patentansprüche

1. Freisetzvorrichtung zum Betätigen eines Einführkatheters (12), welcher eine Außenhülle (16) und einen in der Außenhülle (16) verschiebbar angeordneten Mandrin (14) umfasst,
mit einer länglichen Handhabe (26),
einer an der Handhabe (26) angeordneten Befestigungsaufnahme (46), welche dazu ausgelegt ist, ein proximales Endstück des Mandrins (14) relativ zur Längsachse der Handhabe (26) unverschieblich an der Handhabe (26) festzulegen, und
einer an der Handhabe (26) ausgebildeten Führung (40), welche dazu ausgelegt ist, ein am proximalen Endbereich der Außenhülle (16) angeordnetes Betätigungselement (18, 48) in Bezug auf die Längsachse der Handhabe (26) und auf den Mandrin (14) verschieblich aufzunehmen,
wobei
die Handhabe (26) ein längliches Basisteil (28) und ein mit dem Basisteil (28) verbundenes, entlang der Längsachse des Basisteils (28) verstellbares längliches Verstellteil (30) umfasst, wobei das Verstellteil (30) zwischen einem Justierzustand, in welchem das Verstellteil (30) für eine Längenänderung der Führung (40) axial verschieblich an dem Basisteil (28) gehalten ist, und einem Arretierzustand, in welchem das Verstellteil (30) an dem Basisteil (28) zumindest axial arretiert ist, umstellbar ist.

2. Freisetzvorrichtung nach Anspruch 1,
**dadurch gekennzeichnet,**
**dass** das Verstellteil (30) durch Verdrehen um seine Längsachse zwischen dem Justierzustand und dem Arretierzustand umstellbar ist.

3. Freisetzvorrichtung nach Anspruch 1 oder 2,
**dadurch gekennzeichnet,**
**dass** das Verstellteil (30) und das Basisteil (28) in dem Arretierzustand des Verstellteils (30), nicht jedoch in dem Justierzustand des Verstellteils (30), einander formschlüssig hintergreifen.

4. Freisetzvorrichtung nach einem der vorhergehenden Ansprüche,
**dadurch gekennzeichnet,**
**dass** das Verstellteil (30) lediglich von dem Justierzustand in den Arretierzustand, nicht jedoch von dem Arretierzustand in den Justierzustand umstellbar ist.

5. Freisetzvorrichtung nach einem der vorhergehenden Ansprüche,
**dadurch gekennzeichnet,**
**dass** das Basisteil (28) eine entlang seiner Längsachse verlaufende Verstellteilaufnahme (38) aufweist, in welcher das Verstellteil (30) gehalten ist, wobei die Verstellteilaufnahme (38) und das Verstellteil (30) bevorzugt die Grundform eines Zylinders aufweisen.

6. Freisetzvorrichtung nach Anspruch 5,
**dadurch gekennzeichnet,**
**dass** die Verstellteilaufnahme (38) mehrere insbesondere quer verlaufende, axial beabstandete Sicherungsausnehmungen (60) aufweist, und
**dass** das Verstellteil (30) an seiner Außenseite wenigstens einen Sicherungsvorsprung (62) aufweist, wobei der Sicherungsvorsprung (62) in dem Arretierzustand des Verstellteils (30) mit einer der Sicherungsausnehmungen (60) in Eingriff steht.

7. Freisetzvorrichtung nach Anspruch 5 oder 6,
**dadurch gekennzeichnet,**
**dass** das Verstellteil (30) in dem Arretierzustand und/oder in dem Justierzustand radial an dem Basisteil (28) verrastet ist.

8. Freisetzvorrichtung nach einem der Ansprüche 5 bis 7,
**dadurch gekennzeichnet,**
**dass** die Verstellteilaufnahme (38) wenigstens eine entlang ihrer Längsachse verlaufende Rastnut (66, 66a, 66b) aufweist, und dass das Verstellteil (30) an seiner Außenseite eine Rastnase (64) aufweist, wobei die Rastnase (64) zumindest in dem Arretierzustand in die Rastnut (66, 66a, 66b) eingreift, um das Verstellteil (30) an dem Basisteil (28) radial zu verrasten.

9. Freisetzvorrichtung nach Anspruch 8,
**dadurch gekennzeichnet,**
**dass** die Verstellteilaufnahme (38) eine erste und eine zweite entlang ihrer Längsachse verlaufende Rastnut (66, 66a, 66b) aufweist, wobei die Rastnase (64) in dem Justierzustand mit der ersten Rastnut (66, 66a) und in dem Arretierzustand mit der zweiten Rastnut (66b) in Eingriff steht, und
**dass** die Rastnase (64) und die erste Rastnut (66, 66a) eine jeweilige Anlaufschräge (68a, 68b) aufweisen, und
**dass** die Rastnase (64) und die Rastnuten (66, 66a, 66b) derart zusammenwirken, dass die Rastnase (64) durch ein Umstellen des Verstellteils (30) von dem Justierzustand in den Arretierzustand aufgrund eines Zusammenwirkens der Anlaufschrägen (68a, 68b) außer Eingriff mit der ersten Rastnut (66, 66a), nicht jedoch durch ein Umstellen von dem Arretierzustand in den Justierzustand außer Eingriff mit der zweiten Rastnut (66b) gebracht werden kann.

10. Freisetzvorrichtung nach einem der vorhergehenden Ansprüche,
**dadurch gekennzeichnet,**
**dass** das Betätigungselement (18, 48) durch ein an dem proximalen Endbereich der Außenhülle (16) vorgesehenes Anschlussstück (18) gebildet oder als ein mit dem proximalen Endbereich der Außenhülle (16) koppelbares, separates Element (48) ausgebildet ist.

11. Freisetzvorrichtung nach einem der vorhergehenden Ansprüche,
**dadurch gekennzeichnet,**
**dass** das Betätigungselement (18, 48) drehfest und/oder in axialer Richtung fest mit dem proximalen Endbereich der Außenhülle (16), insbesondere mit einem an dem proximalen Endbereich der Außenhülle (16) vorgesehenen Anschlussstück (18), verbunden oder verbindbar, insbesondere verrastet oder verrastbar, ist.

12. Freisetzvorrichtung nach einem der vorhergehenden Ansprüche,
**dadurch gekennzeichnet,**
**dass** das Betätigungselement (18, 48) zumindest in einer vorgegebenen Axialposition durch Verdrehen um die Längsachse der Handhabe (26) zwischen einer Blockierstellung, in welcher das Betätigungselement (18, 48) in axialer Richtung arretiert ist, und einer Freigabestellung, in welcher das Betätigungselement (18, 48) axial verschieblich in der Führung (40) aufgenommen ist, verstellbar ist.

13. Freisetzvorrichtung nach einem der vorhergehenden Ansprüche,
**dadurch gekennzeichnet,**
**dass** das Basisteil (28) eine im Wesentlichen plane Auflagefläche (36) aufweist.

14. Einführkatheteranordnung, umfassend
einen Einführkatheter (12) mit einer Außenhülle (16) und einen in der Außenhülle (16) verschiebbar angeordneten Mandrin (14), und eine Freisetzvorrichtung (24) zum Betätigen des Einführkatheters (12) nach einem der vorhergehenden Ansprüche.

## Claims

1. A release apparatus for actuating a delivery catheter (12) which comprises an outer sleeve (16) and a mandrin (14) moveably arranged in the outer sleeve (16),
comprising an elongate handle (26);
a fastening receiver (46) arranged at the handle (26) which is configured to fix a proximal end piece of the mandrin (14) to the handle (26) in a non-displaceable manner relative to the longitudinal axis of the handle (26); and a guide (40) formed at the handle (26) which is configured to receive an actuation element (18, 48) displaceably with respect to the longitudinal axis of the handle (26) and with respect to the mandrin (14), said actuation element being arranged at a proximal end region of the outer sleeve (16), wherein the handle (26) has an elongate base part (28) and an elongate adjustment part (30) connected to the base part (28) and adjustable along the longitudinal axis of the base part (28), wherein the adjustment part (30) can be switched between an alignment state, in which the adjustment part (30) is held axially displaceable at the base part (28) for a change of length of the guide (40), and a locking state, in which the adjustment part (30) is at least axially locked to the base part (28).

2. A release apparatus in accordance with claim 1,
**characterized in that**
the adjustment part (30) can be switched between the alignment state and the locking state by rotation about its longitudinal axis.

3. A release apparatus in accordance with claim 1 or claim 2,
**characterized in that**
the adjustment part (30) and the base part (28) engage behind one another in a form-fitting manner in the locking state of the adjustment part (30), but not in the alignment state of the adjustment part (30).

4. A release apparatus in accordance with any one of the preceding claims,
**characterized in that**
the adjustment part (30) can only be switched from the alignment state into the locking state, but not from the locking state into the alignment state.

5. A release apparatus in accordance with any one of the preceding claims,
**characterized in that**
the base part (28) has an adjustment part receiver (38) extending along its longitudinal axis in which the adjustment part (30) is held, wherein the adjustment part receiver (38) and the adjustment part (30) preferably have the basic shape of a cylinder.

6. A release apparatus in accordance with claim 5,
**characterized in that**
the adjustment part receiver (38) has a plurality of axially spaced apart securing recesses (60), in particular extending transversely, and
**in that** the adjustment part (30) has at least one securing projection (62) at its outer side, wherein the securing projection (62) is in engagement with one of the securing recesses (60) in the locking state of the adjustment part (30).

7. A release apparatus in accordance with claim 5 or claim 6,
**characterized in that**
the adjustment part (30) is radially latched to the base part (28) in the locking state and/or in the alignment state.

8. A release apparatus in accordance with any one of the claims 5 to 7,
**characterized in that**
the adjustment part receiver (38) has at least one latching groove (66, 66a, 66b) extending along its longitudinal axis; and
**in that** the adjustment part (30) has a tongue (64) at its outer side, wherein the tongue (64) engages into the latching groove (66, 66a, 66b) at least in the locking state in order to radially lock the adjustment part (30) to the base part (28).

9. A release apparatus in accordance with claim 8,
**characterized in that**
the adjustment part receiver (38) has a first and a second latching groove (66, 66a, 66b) extending along its longitudinal axis, wherein the tongue (64) is in engagement with the first latching groove (66, 66a) in the alignment state and is in engagement with the second latching groove (66b) in the locking state; and
**in that** the tongue (64) and the first latching groove (66, 66a) have a respective run-up chamfer (68a, 68b) and
**in that** the tongue (64) and the latching grooves (66, 66a, 66b) cooperate in such a way that the tongue (64) can be brought out of engagement with the first latching groove (66, 66a) through a switch of the adjustment part (30) from the alignment state into the locking state due to a cooperation of the run-up chamfers (68a, 68b), but cannot be brought out of engagement with the second latching groove (66b) through a switch from the locking state into the alignment state.

10. A release apparatus in accordance with any one of the preceding claims,
**characterized in that**
the actuation element (18, 48) is formed by a connection piece (18) provided at the proximal end region of the outer sleeve (16) or is formed as a separate element (48) that can be coupled to the proximal end region of the outer sleeve (16).

11. A release apparatus in accordance with any one of the preceding claims,
**characterized in that**
the actuation element (18, 48) is rotationally fixedly connected or connectable, in particular latched or latchable, to the proximal end region of the outer sleeve (16), in particular to a connection piece (18) provided at the proximal end region of the outer sleeve (16), and/or the actuation element (18, 48) is fixedly connected or connectable, in particular latched or latchable, in an axial direction to the proximal end region of the outer sleeve (16), in particular to a connection piece (18) provided at the proximal end region of the outer sleeve (16).

12. A release apparatus in accordance with any one of the preceding claims,
**characterized in that**
the actuation element (18, 48) can be moved at least in a predefined axial position by rotation about the longitudinal axis of the handle (26) between a blocking position in which the actuation element (18, 48) is locked in the axial direction and a release position in which the actuation element (18, 48) is received axially movable in the guide (40)

13. A release apparatus in accordance with any one of the preceding claims,
**characterized in that**
the base part (28) has a substantially planar support surface (36).

14. A delivery catheter arrangement comprising
a delivery catheter (12) having an outer sleeve (16) and a mandrin (14) displaceably arranged in the outer sleeve (16) and comprising a release apparatus (24) for actuating the delivery catheter (12) in accordance with any one of the preceding claims.

## Revendications

1. Dispositif de libération pour l'actionnement d'un cathéter d'introduction (12), qui inclut une gaine extérieure (16) et un mandrin (14) agencé avec possibilité de translation dans la gaine extérieure (16), comprenant une manette oblongue (26),
comprenant une réception de fixation (46) agencée sur la manette (26) et conçue pour immobiliser un morceau d'extrémité proximale du mandrin (14) sur la manette (26) sans possibilité de translation par rapport à l'axe longitudinal de la manette (26), et
comprenant un guidage (40) réalisé sur la manette (26), qui est conçu pour recevoir un élément d'actionnement (18, 48) agencé au niveau de la zone d'extrémité proximale de la gaine extérieure (16) avec possibilité de translation par rapport à l'axe longitudinal de la manette (26) et par rapport au mandrin (14),
dans lequel la manette (26) comprend une partie de base oblongue (28) et une partie de réglage oblongue (30) qui est reliée à la partie de base (28) et qui peut être réglée le long de l'axe longitudinal de la partie de base (28), dans lequel la partie de réglage (30) est susceptible d'être inversée entre un état d'ajustement, dans lequel la partie de réglage (30) est retenue sur la partie de base (28) avec possibilité de translation axiale pour une modification de longueur du guidage (40), et un état d'arrêt dans lequel la partie de réglage (30) est arrêtée au moins axialement sur la partie de base (28).

2. Dispositif de libération selon la revendication 1,
**caractérisé en ce que** la partie de réglage (30) est susceptible d'être inversée entre l'état d'ajustement et l'état d'arrêt par rotation autour de son axe longitudinal.

3. Dispositif de libération selon la revendication 1 ou 2,
**caractérisé en ce que** la partie de réglage (30) et la partie de base (28) s'engagent mutuellement par l'arrière en coopération de formes dans l'état d'arrêt de la partie de réglage (30) mais non pas dans l'état d'ajustement de la partie de réglage (30).

4. Dispositif de libération selon la revendication 1 ou 2,
**caractérisé en ce que** la partie de réglage (30) est susceptible d'être inversée uniquement depuis l'état d'ajustement vers l'état d'arrêt, mais non pas depuis l'état d'arrêt vers l'état d'ajustement.

5. Dispositif de libération selon l'une des revendications précédentes, **caractérisé en ce que** la partie de base (28) comprend une réception de partie d'ajustement (38) s'étendant le long de son axe longitudinal, dans laquelle la partie de réglage (30) est maintenue, et dans lequel la réception de partie de réglage (38) et la partie de réglage (30) présentent de préférence la forme de base d'un cylindre.

6. Dispositif de libération selon la revendication 5,
**caractérisé en ce que** la réception de partie de réglage (38) comprend plusieurs réceptions de blocage (60), s'étendant en particulier transversalement et écartées en sens axial, et
**en ce que** la partie de réglage (30) comporte sur sa face extérieure au moins une saillie de blocage (62), ladite saillie de blocage (62) étant en engagement avec l'une des réceptions de blocage (60) dans l'état d'arrêt de la partie de réglage (30).

7. Dispositif de libération selon la revendication 5 ou 6,
**caractérisé en ce que** la partie de réglage (30) est enclenchée radialement sur la partie de base (28) dans l'état d'arrêt et/ou dans l'état d'ajustement.

8. Dispositif de libération selon l'une des revendications 5 à 7, **caractérisé en ce que** la réception de partie de réglage (38) comporte au moins une rainure d'enclenchement (66, 66a, 66b) s'étendant le long de son axe longitudinal, et
**en ce que** la partie de réglage (30) comporte sur sa face extérieure un ergot d'enclenchement (64), ledit ergot d'enclenchement (64) s'engageant dans la rainure d'enclenchement (66, 66a, 66b), au moins dans l'état d'arrêt, afin d'enclencher la partie de réglage (30) radialement sur la partie de base (28).

9. Dispositif de libération selon la revendication 8,
**caractérisé en ce que** la réception de partie de réglage (38) comporte une première et une seconde rainure d'enclenchement (66, 66a, 66b) s'étendant le long de son axe longitudinal, ledit ergot d'enclenchement (64) étant en engagement avec la première rainure d'enclenchement (66, 66a) dans l'état d'ajustement, et avec la seconde rainure d'enclenchement (66b) dans l'état d'arrêt, et
**en ce que** l'ergot d'enclenchement (64) et la première rainure d'enclenchement (66, 66a) comportent un biseau d'approche respectif (68a, 68b), et
**en ce que** l'ergot d'enclenchement (64) et les rainures d'enclenchement (66, 66a, 66b) coopèrent de telle façon que l'ergot d'enclenchement (64) peut être amené hors d'engagement avec la première rainure d'enclenchement (66, 66a) par une inversion de la partie de réglage (30) depuis l'état d'ajustement vers l'état d'arrêt en raison d'une coopération des biseaux d'approche (68a, 68b), mais ne peut pas être amené hors d'engagement avec la seconde rainure d'enclenchement (66b) par une inversion depuis l'état d'arrêt vers l'état d'ajustement.

10. Dispositif de libération selon l'une des revendications précédentes, **caractérisé en ce que** l'élément d'actionnement (18, 48) est formé par une pièce de connexion (18) prévue au niveau de la zone d'extrémité proximale de la gaine extérieure (16), ou est réalisé comme un élément séparé (48) susceptible d'être accouplé avec la zone d'extrémité proximale de la gaine extérieure (16).

11. Dispositif de libération selon l'une des revendications précédentes, **caractérisé en ce que** l'élément d'actionnement (18, 48) est relié ou susceptible d'être relié solidairement en rotation et/ou fermement en direction axiale avec la zone extrémité proximale de la gaine extérieure (16), en particulier avec une pièce de connexion (18) prévue au niveau de la zone d'extrémité proximale de la gaine extérieure (16).

12. Dispositif de libération selon l'une des revendications précédentes, **caractérisé en ce que** l'élément d'actionnement (18, 48) est, au moins dans une position axiale prédéterminée, réglable par rotation autour de l'axe longitudinal de la manette (26) entre une position de blocage dans laquelle l'élément d'actionnement (18, 48) est arrêté en direction axiale, et une position de libération dans laquelle l'élément d'actionnement (18, 48) est reçu avec possibilité de translation axiale dans le guidage (40).

13. Dispositif de libération selon l'une des revendications précédentes, **caractérisé en ce que** la partie de base (28) présente une surface d'appui (36) sensiblement plane.

14. Agencement à cathéter d'introduction, comprenant un cathéter d'introduction (12) avec une gaine extérieure (16) et un mandrin (14) agencé avec possibilité de translation dans la gaine extérieure (16), et un dispositif de libération (24) selon l'une des revendications précédentes pour actionner le cathéter d'introduction (12).
